# EUROPÄISCHE PATENTSCHRIFT

(11) **EP 0 585 682 B1**
(45) Veröffentlichungstag und Bekanntmachung des Hinweises auf die Patenterteilung: **06.10.1999**
(21) Anmeldenummer: 93112933.2
(22) Anmeldetag: 12.08.1993
(51) Int. Cl.: C07D 295/02, C07D 295/10, C07D 295/08

(54) **Verfahren zur Herstellung von 2,6-Dimethylmorpholin aus N-(2-Hydroxypropyl)-2,6-dimethylmorpholin**
Process for the preparation of 2,6-dimethylmorpholine from N-(2-hydroxypropyl)-2,6-dimethylmorpholine
Procédé pour la préparation de 2,6-deméthylmorpholine à partir ou N-(2-hydroxypropyl)-2,6-diméthylmorpholine

(30) Priorität: 29.08.1992 DE 4228885
(43) Veröffentlichungstag der Anmeldung: 09.03.1994
(73) Patentinhaber: BASF Aktiengesellschaft, 67063 Ludwigshafen (DE)
(72) Erfinder: Pinkos, Rolf, Dr., D-6702 Bad Duerkheim (DE); Merkle, Hans Rupert, Dr., D-6700 Ludwigshafen (DE); Fischer, Rolf, Dr., D-6900 Heidelberg (DE)

(56) Entgegenhaltungen:
- EP-A- 0 007 520
- EP-A- 0 026 367
- EP-A- 0 070 397
- EP-A- 0 094 565
- EP-A- 0 129 904
- EP-A- 0 142 742
- EP-A- 0 283 648
- DE-C- 650 380
- US-A- 3 083 202
- US-A- 3 151 112
- US-A- 3 413 292

## Beschreibung

Die vorliegende Erfindung betrifft Verfahren zur Herstellung von 2,6-Dimethylmorpholin aus N-(2-Hydroxypropyl)-2,6-dimethylmorpholin durch Dehydrierung bzw. durch Wasserabspaltung und anschließender Hydrolyse.

2,6-Dimethylmorpholin und das nicht vorbeschriebene N-(2-Oxopropyl)-2,6-dimethylmorpholin sind gesuchte Zwischenverbindungen für Synthesen z.B. für Pflanzenschutz- bzw. Pharmawirkstoffe.

Aus der EP-A-94 565 ist ein Verfahren zur Herstellung von 2,6-Dimethylmorpholin bekannt, in dem Bis-(2-hydroxypropyl)-amin mit überstöchiometrischen Mengen Schwefelsäure gewonnen wird. Für die Freisetzung des 2,6-Dimethylmorpholins muß die Schwefelsäure neutralisiert werden, wobei ein Zwangsanfall an Sulfaten resultiert.

Der vorliegenden Erfindung lag daher die Aufgabe zugrunde, dem zuvor genannten Nachteil abzuhelfen.

Demgemäß wurde ein neues und verbessertes Verfahren zur Herstellung von 2,6-Dimethylmorpholin aus N-(2-Hydroxypropyl)-2,6-dimethylmorpholin gefunden, welches dadurch gekennzeichnet ist, daß man N-(2-Hydroxypropyl)-2,6-dimethylmorpholin
a) in Gegenwart von Katalysatoren der I., II. und/oder VIII. Nebengruppe des Periodensystems der Elemente bei Temperaturen von 150 bis 600°C und anschließend das gebildete N-(2-Oxopropyl)-2,6-dimethylmorpholin in gereinigter Form oder direkt mit Wasser bei Temperaturen von 30 bis 300°C umsetzt oder
b) in Gegenwart von sauren Katalysatoren bei Temperaturen von 100 bis 400°C umsetzt und anschließend mit Wasser bei Temperaturen von 30 bis 300°C sauer hydrolysiert.

Ferner wurde eine neue Verbindung, das N-(2-Oxopropyl)-2,6-dimethylmorpholin, gefunden.

Die erfindungsgemäßen Verfahren lassen sich wie folgt durchführen:

Es können jeweils das cis- oder trans-N-(2-Hydroxypropyl)-2,6-dimethylmorpholin oder bevorzugt deren Isomerengemische eingesetzt werden, die bei der Cyclisierung von Tris-(2-hydroxypropyl)amin, einem Nebenprodukt (Abfallprodukt) bei der Bis-(2-hydroxypropyl)aminherstellung, erhalten werden.
a) N-(2-Hydroxypropyl)-2,6-dimethylmorpholin kann bei Temperaturen von 150 bis 600°C in der Flüssig- oder Gasphase, bevorzugt bei Temperaturen von 280 bis 400°C in der Gasphase und Drücken von 0,001 bis 5 bar, bevorzugt 0,02 bis 2 bar, besonders bevorzugt bei Normaldruck (Atmosphärendruck) zum N-(2-Oxopropyl)-2,6-dimethylmorpholin oxidiert werden.
   Die Oxidation von N-(2-Hydroxypropyl)-2,6-dimethylmorpholin zu N-(2-Oxopropyl)-2,6-dimethylmorpholin kann mit üblichen Oxidationsmitteln, wie sie in Houben-Weyl Band VII/2a (1973) beschrieben bzw. zitiert sind, z.B. Mangandioxid oder Chrom-IV-Verbindungen durchgeführt werden. Bevorzugt sind Katalysatoren für katalytische Dehydrierungen wie Elemente der I., II. und/oder VIII. Nebengruppe des Periodensystems der Elemente in metallischer Form, besonders bevorzugt in oxidischer Form, oder Kombinationen aus den Metallen mit Metalloxiden.
   Als Katalysatoren der I., II. und/oder VIII. Nebengruppe des Periodensystems der Elemente in der metallischen Form eignen sich Kupfer, Silber, Gold, Zink, Gadmium, Eisen, Cobalt, Nickel, Ruthenium, Rhodium, Palladium, Osmium, Iridium und Platin, bevorzugt Kupfer, Silber, Gold, Ruthenium, Rhodium, Palladium, Osmium, Iridium und Platin, besonders bevorzugt Kupfer, Silber und Zink.
   Als Katalysatoren der I., II. und/oder VIII. Nebengruppe des Periodensystems der Elemente in der oxidischen Form eignen sich Kupfer, Silber, Gold, Zink, Cadmium, Eisen, Cobalt, Nickel, Ruthenium, Rhodium, Palladium, Osmium, Iridium und Platin, bevorzugt Kupfer, Silber, Gold, Zink,Ruthenium, Rhodium, Palladium, Osmium, Iridium und Platin, besonders bevorzugt Kupfer, Silber und Zink.
   Die Reaktion kann mit oder ohne Trägergas durchgeführt werden. Bevorzugt sind inerte Trägergase wie Wasserstoff, Stickstoff oder Argon. Gegebenenfalls kann Sauerstoff oder Luft bis zu einem Anteil von beispielsweise 5 % zum Trägergas zudosiert werden.
   N-(2-Oxopropyl)-2,6-dimethylmorpholin kann destillativ von nicht umgesetzten N-(2-Hydroxypropyl)-2,6-dimethylmorpholin und Nebenprodukten getrennt werden.
   Das N-(2-Oxopropyl)-2,6-dimethylmorpholin kann in gereinigter Form oder direkt aus der Dehydrierung mit Wasser bei Temperaturen von 30 bis 300°C, bevorzugt 100 bis 250°C und Drücken von 0,01 bis 50 bar, bevorzugt 0,5 bis 20 bar, besonders bevorzugt 1 bis 10 bar zu 2,6-Dimethylmorpholin hydrolysiert werden.
   Dabei kann in Gegenwart üblicher saurer oder alkalischer Katalysatoren wie verdünnter Phosphor- oder Schwefelsäure oder verdünnter Natronlauge oder Kalilauge gearbeitet werden. Besonders bevorzugt wird die Reaktion aber autokatalytisch, d.h. ohne Zusatz von Katalysatoren, durchgeführt.
   Das Molverhältnis von Wasser zum N-(2-oxopropyl)-2,6-dimethylmorpholin kann in weiten Grenzen variiert werden wie 500 : 1 bis 0,5 : 1, bevorzugt 50 : 1 bis 1 : 1, besonders bevorzugt 10 : 1 bis 2 : 1.
   Bei kontinuierlicher Durchführung kann Wasser zusammen mit 2,6-Dimethylmorpholin, gegebenenfalls unter Druck abdestilliert werden.
   Bei unvollständigem Umsatz kann das N-(2-Oxopropyl)-2,6-dimethylmorpholin destillativ von 2,6-Dimethylmorpholin abgetrennt und wieder eingesetzt werden.
b) N-(2-Hydroxypropyl)-2,6-dimethylmorpholin kann durch sauer katalysierte Wasserabspaltung in der Flüssigphase, bevorzugt in der Gasphase, an sauren Katalysatoren bei Temperaturen von 100 bis 400°C, bevorzugt 150 bis 350°C, besonders bevorzugt bei 180 bis 290°C und Drücken von 0,01 und 2 bar, bevorzugt sind 0,05 bis 1 bar zu N-(2-Propenyl)-2,6-dimethylmorpholin und N-(1-Propenyl)-2,6-dimethylmorpholin dehydratisiert werden. Die Verweilzeit kann bei den Reaktionen in der Gasphase z.B. 1 bis 5 Sekunden betragen.
   Dem Feed kann Wasser zugesetzt werden, z.B. 0 bis 10, bevorzugt 0,01 bis 1 Mol/Mol N-(2-Hydroxypropyl)-2,6-dimethylmorpholin.
   Als saure Katalysatoren eignen sich saure oder supersaure Metalloxide der I., II., IV. und VIII. Nebengruppe des Periodensystems der Elemente, bevorzugt TiO₂, ZrO₂, Fe₂O₃ und ZnO oder der II., III. und IV Hauptgruppe des Periodensystems der Elemente, bevorzugt MgO, B₂O₃, Al₂O₃, SiO₂ und SnO₂ oder Kombinationen derselben, wie z.B. TiO₂/ZnO oder Al₂O₃/MgO. Zur Erhöhung der Säurestärke können die Oxide mit Säuren wie z.B. Schwefelsäure oder Phosphorsäure behandelt werden.
   Als saure Katalysatoren eignen sich ferner saure Zeolithe, beispielsweise Vertreter aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe gehören auch die sogenannten "ultrastabilen" Zeolithe des Faujasittyps, d.h. dealuminierte Zeolithe.
   Geeignet sind auch Zeolithe mit Pentasilstruktur wie ZSM-5, ZSM-11 und ZBM-10. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.
   Ebenso geeignet sind auch Schichtsilikate wie z.B. Tonsil, die mit Aluminiumoxid dotiert sein können oder Alumosilikate.
   Weiterhin sind Heteropolysäuren als saure Katalysatoren geeignet. Dies sind anorganische Polysäuren, die im Gegensatz zu Isopolysäuren mindestens zwei verschiedene Zentralatome besitzen. Als Beispiele seien Dodecawolframphosphorsäure H₃PW₁₂O₄₀ x H₂O, Dodecamolybdophosphorsäure H₃PMo₁₂O₄₀ x H₂O genannt. Prinzipiell können die in EP-A-158 229 genannten Katalysatoren und Katalysatorkombinationen eingesetzt werden.
   Bevorzugte Heteropolysäuren sind Heteropolysäuren von Molybdän oder Wolfram mit Phosphorsäure, Tellursäure, Selensäure, Arsensäure, Kieselsäure, insbesondere mit Phosphorsäure.
   Die Protonen der Heteropolysäuren können teilweise durch Metallionen ersetzt sein, bevorzugt sind dabei die Alkali- und Erdalkalimetallionen.
   Grundsätzlich sind Kombinationen aus den genannten Katalysatoren geeignet, z.B. Kombinationen aus Aluminiumoxiden, die durch Säuren wie Schwefel- oder Phosphorsäure modifiziert wurden und Heteropolysäuren.
   Die sauren Katalysatoren können auch mit Metallen der VIII. Nebengruppe der Elemente, z.B. Pd dotiert sein.
   Die Reaktionsausträge können destillativ in Wasser, N-(2-Propenyl)-2,6-dimethylmorpholin, 2,6-Dimethylmorpholin und eventuell nicht umgesetztes N-(2-Hydroxynropyl)-2,6-dimethylmorpholin sowie Propionaldehyd bzw. dessen Folgeprodukte aufgetrennt werden.
   Besonders bevorzugt werden die Reaktionsausträge mit einem Isomerisierungskatalysator für die Isomerisierung von N-(2-Propenyl)-2,6-dimethylmorpholin zu N-(1-Propenyl)2,6-dimethylmorpholin und, gegebenenfalls kontinuierlich, mit Wasser versetzt und auf 25 bis 250°C, bevorzugt 100 bis 200°C erwärmt.
   Diskontinuierlich kann man in einem Druckgefäß arbeiten, in dem sich der Eigendruck einstellt, oder unter Destillationsbedingungen, gegebenenfalls unter einem Druck bis 20 bar, wobei gebildetes 2,6-Dimethylmorpholin abdestilliert wird. Die zugesetzte Wassermenge kann 0,5 bis 10 Mol-Äquivalente bzgl. eingesetztem N-(2-Hydroxypropyl)-2,6-dimethylmorpholin betragen. Ein geeigneter Isomerisierungskatalysator ist beispielsweise Pd auf Aktivkohle, der in einem Gewichtsverhältnis zu eingesetztem N-(2-Hydroxypropyl)-2,6-dimethylmorpholin von 0,001 bis 0,3 zugesetzt werden kann.
   Die Wasserabspaltung kann z. B. in Wirbelbett- oder bevorzugt in Festbettreaktoren durchgeführt werden.
   Das Verfahren wird anhand der nachstehenden Beispiele näher erläutert (sofern nicht anders angegeben, sind die Prozentangaben Gew.%, bestimmt nach Gaschromatographie mit innerem Standard). Eingesetzt wurden an N-(2-Oxopropyl)-2,6-dimethylmorpholin bzw. N-(2-Hydroxypropyl)-2,6-dimethylmorpholin jeweils die cis-, trans-Isomerengemische (cis:trans = 60 bis 80:40 bis 20).

### Beispiele

### zu a)

Die Verfahrensvariante a) läuft zweistufig ab. Die Beispiele 1 bis 3 beschreiben die erste Stufe, die Dehydrierung; das Beispiel 4 die zweite Stufe, die Hydrolyse.

### Beispiel 1

An 10 g eines strangförmigen Katalysators (ca. 94 % ZnO, 4 % MgO, Rest CaO und Na₂O) wurden 7 g N-(2-Hydroxypropyl)-2,6-dimethylmorpholin/h in einem N₂-Strom (4 l/h) umgesetzt. Bei 300°C enthielt der Austrag 7 % N-(2-Oxopropyl)-2,6-dimethylmorpholin, 86,4 % N-(2-Hydroxypropyl)-2,6-dimethylmorpholin, 1,4 % N-(2-Propenyl)-2,6-dimethylmorpholin und 2,1 % 2,6-Dimethylmorpholin. Bei 350°C enthielt der Austrag 15,3 % N-(2-Oxopropyl)-2,6-dimethylmorpholin, 75,3 % N-(2-Hydroxypropyl)-2,6-dimethylmorpholin, 3,4 % N-(2-Propenyl)-2,6-dimethylmorpholin und 2,8 % 2,6-Dimethylmorpholin. Bei 380°C fanden sich 23,8 % N-(2-Oxopropyl)-2,6-dimethylmorpholin, 61,1 % N-(2-Hydroxypropyl)-2,6-dimethylmorpholin, 4,8 % N-(2-Propenyl)-2,6-dimethylmorpholin und 4,9 % 2,6-Dimethylmorpholin im Austrag.

Die cis:trans-Isomerenverhältnisse der Morpholinderivate betrugen jeweils ca. 7:3.

### Beispiel 2

An 180 g eines strangförmigen ZnO-Katalysators wurden 15 g N-(2-Hydroxypropyl)-2,6-dimethylmorpholin/h bei 250 bis 400°C und einem N₂-Strom von 18 1/h umgesetzt. Nach 1-2 Stunden betrugen die Austräge ca. 15 g/h. Die Ergebnisse sind in folgender Tabelle zusammengefaßt:

| Temp. [°C] | NODM [%] | NHDM [%] | N-2-PM [%] | 1,6-DMM [%] |
|---|---|---|---|---|
| 250 *) | 33,9 | 41,9 | 4,5 | 3,3 |
| 290 | 33,9 | 54,4 | 2,8 | 0,7 |
| 320 | 53,6 | 26,3 | 5,9 | 2,2 |
| 350 | 70,2 | 15,4 | 4,1 | 1,2 |
| 370 | 72,4 | 5,2 | 3,8 | 2,5 |
| 400 | 73,0 | 1,6 | 3,7 | 2,8 |

| | | | | |
|---|---|---|---|---|
| NODM = N-(2-Oxopropyl)-2,6-dimethylmorpholin; | | | | |
| NHDM = N-(2-Hydroxypropyl)-2,6-dimethylmorpholin; | | | | |
| N-2-PM = N-(2-Propenyl)-2,6-dimethylmorpholin; | | | | |
| 1,6-DMM = 2,6-Dimethylmorpholin; | | | | |
| *) nicht erfindungsgemäßes Beispiel | | | | |

### Beispiel 3

Analog Beispiel 2 wurden über 23 h Stunden 15 g/h N-(2-Hydroxypropyl)-2,6-dimethylmorpholin an 142 g ZnO bei 350°C umgesetzt. In nachfolgender Tabelle sind einige Ergebnisse aufgeführt:

| Zeit [h] | NODM [%] | NHDM [%] | N-2-PM [%] | 1,6-DMM [%] | NODM-Selekt. |
|---|---|---|---|---|---|
| 3 | 65,7 | 2,4 | 5,6 | 8,3 | 67 |
| 14 | 76,5 | 9,9 | 3,1 | 1,8 | 85 |
| 23 | 74,0 | 13,2 | 2,7 | 1,4 | 85 |

Die gesammelten Austräge (340 g) wurden über eine Füllkörperkolonne (10 theoretische Boden) fraktioniert destilliert. Die isomeren N-(2-Oxopropyl)-2,6-dimethylmorpholine sieden bei 1 mbar zwischen 86 und 90°C.

N-(2-Oxopropyl)-2,6-dimethylmorpholin ist durch Massenspektrum, ¹H-und ¹³C-NMR sowie Infrarot-Spektum eindeutig charakterisiert.

Als Beispiel seien die IR-Daten der Oxopropyle aufgeführt:

cis-N-(2-Oxopropyl)-2,6-dimethylmorpholin: 2981, 2942, 2871, 2816, 1729, 1452, 1359, 1323, 1219, 1151, 1082, 970, 879, 847, 775 cm-¹. trans-N-(2-oxopropyl)-2,6-dimethylmorpholin: 2981, 2942, 2896, 2816, 1730, 1461, 1382, 1227, 1153, 1131, 1076, 1036, 973, 878, 850, 800 cm⁻¹.

### Beispiel 4

0,5 g N-(2-Oxopropyl)-2,6-dimethylmorpholin und 3 g Wasser werden 75 Minuten in einem Autoklaven auf 150°C erhitzt. Nach Abkühlen wurden 17,5 Mol-% 2,6-Dimethylmorpholin und ca. 81 Mol-% Edukt gefunden.

### zu b)

### Beispiel 5

28 ml N-(2-Hydroxypropyl)-2,6-dimethylmorpholin wurden in 3 Stunden in einem Vorverdampfer in einem Stickstoffstrom (14 ml/h, 250°C) verdampft und über SiO₂-Stränge (3 mm) geleitet. Bei 250°C Reaktionstemperatur fanden sich im zweiphasigen Reaktionsaustrag (21 g) 56 % N-(2-Propenyl)-2,6-dimethylmorpholin, 3 % 2,6-Dimethylmorpholin, 15 % N-(1-Propenyl)-2,6-dimethylmorpholin und 0,8 % Edukt. Der Rest bestand im wesentlichen aus Wasser.

5 g dieses Austrages wurden mit 1,5 g Wasser und 0,3 g Pd (10 %)/Aktivkohle versetzt und 1 h im Druckgefäß auf 150°C (5 bar Eigendruck) erhitzt. Es fanden sich 20 % 2,6-Dimethylmorpholin und 1,8 % N-(2-Propenyl)-2,6-dimethylmorpholin.

### Beispiel 6

An 200 g Al₂O₃ wurden innerhalb 5 Stunden 34,2 g N-(2-Hydroxypropyl)-2,6-dimethylmoprholin bei 50 mbar und 200°C Reaktionstemperatur umgesetzt. Der Reaktionsaustrag (33,3 g ) wurde mit 2 g Pd (10 %)/C und 10 g Wasser versetzt und 2 h auf 150°C erhitzt. Es fanden sich 27 % 2,6-Dimethylmorpholin neben geringen Mengen N-(2-Propenyl)-2,6-dimethylmorpholin.

## Patentansprüche

1. Verfahren zur Herstellung von 2,6-Dimethylmorpholin aus N-(2-Hydroxypropyl)-2,6-dimethylmorpholin, dadurch gekennzeichnet, daß man N-(2-Hydroxypropyl)-2,6-dimethylmorpholin
a) in Gegenwart von Katalysatoren der I., II. und/oder VIII. Nebengruppe des Periodensystems der Elemente bei Temperaturen von 280 bis 400°C und anschließend das gebildete N-(2-Oxopropyl)-2,6-dimethylmorpholin in gereinigter Form oder direkt mit Wasser bei Temperaturen von 30 bis 300°C umsetzt oder
b) in Gegenwart von sauren Katalysatoren bei Temperaturen von 100 bis 400°C umsetzt und anschließend mit Wasser bei Temperaturen von 30 bis 300°C hydrolysiert.

2. Verfahren zur Herstellung von 2,6-Dimethylmorpholin aus N-(2-Hydroxypropyl)-2,6-dimethylmorpholin nach Anspruch 1a, dadurch gekennzeichnet, daß man als Katalysatoren der I., II. und/ oder VIII. Nebengruppe des Periodensystems der Elemente Oxide und/oder Metalle der Elemente Kupfer, Silber, Gold, Zink, Cadmium, Eisen, Cobalt, Nickel, Ruthenium, Rhodium, Palladium, Osmium, Iridium und/oder Platin einsetzt.

3. Verfahren zur Herstellung von 2,6-Dimethylmorpholin aus N-(2-Hydroxypropyl)-2,6-dimethylmorpholin nach Anspruch 1a, dadurch gekennzeichnet, daß man als Katalysatoren der I., II. und/ oder VIII. Nebengruppe des Periodensystems der Elemente Oxide und/oder die Metalle der Elemente Kupfer, Silber und Zink einsetzt.

4. Verfahren zur Herstellung von 2,6-Dimethylmorpholin aus N-(2-Hydroxypropyl)-2,6-dimethylmorpholin nach Anspruch 1b, dadurch gekennzeichnet, daß man als saure Katalysatoren Metalloxide, Zeolithe, Schichtsilikate oder Heteropolysäuren einsetzt.

5. Verfahren zur Herstellung von 2,6-Dimethylmorpholin aus N-(2-Hydroxypropyl)-2,6-dimethylmorpholin, dadurch gekennzeichnet, daß man N-(2-Hydroxypropyl)-2,6-dimethylmorpholin in Gegenwart von sauren Katalysatoren bei Temperaturen von 100 bis 400°C umsetzt und anschließend mit Wasser in Gegenwart von Isomerisierungskatalysatoren bei Temperaturen von 25 bis 250°C hydrolysiert.

6. Verfahren gemäß Anspruch 5, dadurch gekennzeichnet, daß die Hydrolyse in Gegenwart von Pd auf Aktivkohle bei 25 bis 250°C durchgeführt wird.

7. Verfahren zur Herstellung von 2,6-Dimethylmorpholin aus N-(2-Hydroxypropyl)-2,6-dimethylmorpholin nach den Ansprüchen 1b und 4, dadurch gekennzeichnet, daß die sauren Katalysatoren Metalle der VIII. Nebengruppe des Periodensystems der Elemente enthalten.

8. N-(2-Oxopropyl)-2,6-dimethylmorpholin.

## Claims

1. A process for preparing 2,6-dimethylmorpholine from N-(2-hydroxypropyl)-2,6-dimethylmorpholine, which comprises reaction of N-(2-hydroxypropyl)-2,6-dimethylmorpholine
a) in the presence of catalysts of group Ib, IIb and/or VIII of the Periodic Table of the Elements at temperatures from 280 to 400°C and then of the produced N-(2-oxopropyl)-2,6-dimethylmorpholine in purified form or directly with water at temperatures from 30 to 300°C, or
b) in the presence of acidic catalysts at temperatures from 100 to 400°C, and subsequent hydrolysis with water at temperatures from 30 to 300°C.

2. A process for preparing 2,6-dimethylmorpholine from N-(2-hydroxypropyl)-2,6-dimethylmorpholine as claimed in claim 1a, wherein the catalysts of group Ib, IIb and/or VIII of the Periodic Table of the Elements employed are oxides and/or metals of the elements copper, silver, gold, zinc, cadmium, iron, cobalt, nickel, ruthenium, rhodium, palladium, osmium, iridium and/or platinum.

3. A process for preparing 2,6-dimethylmorpholine from N-(2-hydroxypropyl)-2,6-dimethylmorpholine as claimed in claim 1a, wherein the catalysts of group Ib, IIb and/or VIII of the Periodic Table of the Elements employed are oxides and/or metals of the elements copper, silver and zinc.

4. A process for preparing 2,6-dimethylmorpholine from N-(2-hydroxypropyl)-2,6-dimethylmorpholine as claimed in claim 1b, wherein metal oxides, zeolites, sheet silicates or heteropolyacids are employed as acidic catalysts.

5. A process for preparing 2,6-dimethylmorpholine from N-(2-hydroxypropyl)-2,6-dimethylmorpholine, which comprises reaction of N-(2-hydroxypropyl)-2,6-dimethylmorpholine in the presence of acidic catalysts at temperatures from 100 to 400°C, and subsequent hydrolysis with water in the presence of isomerization catalysts at temperatures from 25 to 250°C.

6. A process as claimed in claim 5, wherein the hydrolysis is carried out in the presence of Pd on activated carbon at 25 to 250°C.

7. A process for preparing 2,6-dimethylmorpholine from N-(2-hydroxypropyl)-2,6-dimethylmorpholine as claimed in claims 1b and 4, wherein the acidic catalysts comprise metals of group VIII of the Periodic Table of the Elements.

8. N-(2-Oxopropyl)-2,6-dimethylmorpholine.

## Revendications

1. Procédé pour la préparation de 2,6-diméthylmorpholine à partir de N-(2-hydroxypropyl)-2,6-diméthylmorpholine, caractérisé par le fait qu'on fait réagir de la N-(2-hydroxypropyl)-2,6-diméthylmorpholine
a) en présence de catalyseurs du sous-groupe I, II et/ou VIII de la classification périodique des éléments à des températures de 280 à 400°C et on fait ensuite réagir la N-(2-oxo-propyl)-2,6-diméthylmorpholine formée, sous forme purifiée ou directement avec de l'eau à des températures de 30 à 300°C, ou
b) en présence de catalyseurs acides à des températures de 100 à 400°C et on hydrolyse ensuite avec de l'eau à des températures de 30 à 300°C.

2. Procédé pour la préparation de 2,6-diméthylmorpholine à partir de N-(2-hydroxpropyl)-2,6-diméthylmorpholine selon la revendication 1a, caractérisé par le fait qu'on utilise comme catalyseurs du sous-groupe I, II et/ou VIII de la classification périodique des éléments des oxydes et/ou des métaux des éléments cuivre, argent, or, zinc, cadmium, fer, cobalt, nickel, ruthénium, rhodium, palladium, osmium, iridium et/ou platine.

3. Procédé pour la préparation de 2,6-diméthylmorpholine à partir du Composé N-(2-hydroxypropyl)-2,6-diméthylmorpholine selon la revendication 1a, caractérisé par le fait qu'on utilise comme catalyseurs du sous-groupe I, II et/ou VIII de la classification périodique des éléments des oxydes et/ou des métaux des éléments cuivre, argent et zinc.

4. Procédé pour la préparation de 2,6-diméthylmorpholine à partir de N-(2-hydroxypropyl)-2,6-diméthylmorpholine selon la revendication 1b, caractérisé par le fait qu'on utilise comme catalyseurs acides des oxydes métalliques, des zéolites, des silicates lamellaires ou des hétéropolyacides.

5. Procédé pour la préparation de 2,6-diméthylmorpholine à partir de N-(2-hydroxypropyl)-2,6-diméthylmorpholine, caractérisé par le fait qu'on fait réagir de la N-(2-hydroxypropyl)-2,6-diméthylmorpholine en présence de catalyseurs acides à des températures de 100 à 400°C et on hydrolyse ensuite avec de l'eau, en présence de catalyseurs d'isomérisation, à des températures de 25 à 250°C.

6. Procédé selon la revendication 5, caractérisé par le fait qu'on effectue l'hydrolyse en présence de Pd sur du charbon actif entre 25 et 250°C.

7. Procédé pour la préparation de 2,6-diméthylmorpholine à partir de N-(2-hydroxypropyl)-2,6-diméthylmorpholine selon les revendications 1b et 4, caractérisé par le fait que les catalyseurs acides contiennent des métaux du sous-groupe VIII de la classification périodique des éléments.

8. N-(2-oxopropyl)-2,6-diméthylmorpholine.
